# EUROPÄISCHE PATENTSCHRIFT

(11) **EP 0 599 433 B1**
(45) Veröffentlichungstag und Bekanntmachung des Hinweises auf die Patenterteilung: **07.01.1998**
(21) Anmeldenummer: 93250301.4
(22) Anmeldetag: 03.11.1993
(51) Int. Cl.: A61K 7/32

(54) **Desodorierende Wirkstoffe**
Deodorant agents
Agents désodorisants

(30) Priorität: 26.11.1992 DE 4240674
(43) Veröffentlichungstag der Anmeldung: 01.06.1994
(73) Patentinhaber: Schülke & Mayr GmbH, 22851 Norderstedt (DE)
(72) Erfinder: Beilfuss, Wolfgang, Dr., D-22339 Hamburg (DE); Diehl, Karl-Heinz, D-22844 Norderstedt (DE); Eggensperger, Heinz, Dr., D-22397 Hamburg (DE); Oltmanns, Peter, Dr., D-20255 Hamburg (DE)
(74) Vertreter: UEXKÜLL & STOLBERG

(56) Entgegenhaltungen:
- EP-A- 0 373 661
- EP-A- 0 524 548
- EP-A- 0 547 727
- EP-A- 0 550 960
- DE-A- 3 017 221

## Beschreibung

Die Erfindung betrifft desodorierend wirkende Verbindungen und insbesondere deren Verwendung zur Unterbindung von durch Mikroorganismen verursachten unangenehmen Gerüchen.

Körpergeruch entsteht, wenn der an sich geruchlose Schweiß durch Mikroorganismen zersetzt wird. Aus den Schweißinhaltsstoffen, dem Hauttalg und Hautzellresten vermögen die auf der Haut anzutreffenden, vor allem gram-positiven Keime Stoffe zu bilden, die einen unangenehmen Geruch aufweisen. Diese mikrobiell erzeugten Stoffe verbreiten nicht nur schlechten Geruch, sondern können auch hautirritierend wirken.

Zur Vermeidung von unangenehmem Körpergeruch sind beispielsweise folgende Möglichkeiten (Wirkprinzipien) bekannt:
a) Durch Adstringentien, insbesondere vorwiegend Aluminiumsalze wie Aluminiumhydroxychlorid kann die Absonderung von Schweiß verringert oder unterbunden werden. Adstringentien denaturieren die Hautproteine und greifen drastisch in den Wärmehaushalt der Achselregion ein.
b) Durch antimikrobielle Stoffe kann die Bakterienflora auf der Haut Verringert werden. Dabei sollten im Idealfall nur die den Geruch verursachenden Mikroorganismen vernichtet werden. Meistens wird aber die gesamte Mikroflora der Haut geschädigt. Gelegentlich werden die Mikroben, die keinen Geruch verursachen, sogar stärker geschädigt.
c) Durch Duftstoffe kann Körpergeruch überdeckt werden. Zuweilen kann aber die Mischung aus Körpergeruch und Parfümduft ebenfalls eher unangenehm riechen.

Zur Verringerung der Bildung von durch Mikroorganismen verursachtem unangenehmen Geruch mittels antimikrobieller Stoffe ist beispielsweise die Verwendung von 5-Chlor-2-(2,4-dichlorphenoxy)phenol (Irgasan® DP 300) bzw. Farnesol in Kombination mit Phenoxyethanol bzw. gegebenenfalls in Gegenwart von Glycerinmonolaurat als Deowirkstoff bekannt. Die Verwendung dieser bekannten Wirkstoffkombinationen hat jedoch Nachteile. Irgasan® DP 300 ist zwar sehr gut wirksam, enthält jedoch organisch gebundenes Halogen und ist daher wenig umweltverträglich, da bei der Herstellung bzw. bei der thermischen Zersetzung die Bildung von hochtoxischen Dioxinen nicht auszuschließen ist. Farnesol in Kombination mit Phenoxyethanol und/oder Glycerinmonolaurat besitzt eine nicht in allen Fällen ausreichende Wirksamkeit gegen die geruchsverursachenden Mikroorganismen. Ferner besitzt diese Wirkstoffkombination eher unspezifische Wirkung gegen gram-positive und gram-negative Bakterien.

Die der Erfindung zugrundeliegende Aufgabe besteht darin, diese Nachteile zu vermeiden und neue desodorierende Wirkstoffe (DeoWirkstoffe) oder Wirkstoffkombinationen zur Verfügung zu stellen, die insbesondere gegen die im Hinblick auf Geruchsverursachung besonders relevanten gram-positiven Bakterien eine gute Wirkung zeigen, selektiv gegen geruchsverursachende Mikroorganismen unter weitgehender Schonung der natürlichen Hautflora wirken, ein gutes Haftvermögen auf der Haut und ausgezeichnete Stabilität besitzen und darüber hinaus geruchsarm bzw. geruchsneutral sind.

Zur Lösung der erfindungsgemäßen Aufgabe wird vorgeschlagen, Glycerinmonoalkylether der allgemeinen Formel

R-O-CH₂-CHOH-CH₂OH,

in der R eine verzweigte oder unverzweigte C₆-C₁₈-Alkylgruppe ist, wobei die Alkylgruppe mit einer oder mehreren Hydroxy- und/oder C₁-C₄-Alkoxygruppe(n) substituiert und/oder die Alkylkette durch bis zu vier Sauerstoffatome unterbrochen sein kann, d.h. Alkylenoxygruppen wie Ethylenoxy- und Propylenoxygruppen enthalten kann, einzeln oder in Form eines Gemisches aus zwei oder mehr derselben als Deowirkstoff zur Unterbindung von unangenehmen Gerüchen zu verwenden, ausgenommen Kombinationen von 1-Monoisostearylglycerylether mit Glycerylmonooleat oder Glycerylmonolaurat.

Bevorzugte Ausführungsformen sind Gegenstand der Unteransprüche.

Es wurde überraschenderweise gefunden, daß 3-(Alkyloxy)-propan-1,2-diole (Glycerinmonoalkylether) der Formel

R - O - CH₂ - CHOH - CH₂OH

eine gute desodorierende Wirkung besitzen.

Vorzugsweise ist der Rest R eine C₈-C₁₂-Alkylgruppe und insbesondere eine 2-Ethylhexylgruppe oder Dodecylgruppe, wovon 2-Ethylhexyl am meisten bevorzugt ist. 2-Ethylhexylglycerinether in einer spezifisch geruchsarmen und peroxidarmen Qualität ist besonders bevorzugt.

Es wurde ferner gefunden, daß ein Gemisch aus zwei oder mehreren dieser Glycerinmonalkylether vorteilhaft verwendet werden kann, wobei Gemische aus 2-Ethylhexylglycerinether und Dodecylglycerinether bevorzugt sind.

Es wird angenommen, daß die überraschende desodorierende Wirkung der erfindungsgemäßen Glycrinmonoalkylether auf mehreren unterschiedlichen Wirkungsmechanismen beruhen kann.
1) Die Glycerinether wirken antimikrobiell, wobei insbesondere bei gram-positiven, geruchsverursachenden Bakterien eine gute Wirkung erzielt wird.
2) Die Glycerinether hemmen Enzyme oder Katalysatoren, die für die Geruchsbildung aus dem primär geruchlosen Schweiß verantwortlich sind.
3) Die Glycerinether üben einen Einfluß auf die Schweißdrüsensekretion aus, wobei die Schweißabsonderung reduziert wird.
4) Die Glycerinether überdecken oder absorbieren die geruchsintensiven Schweißbestandteile oder treten in Wechselwirkung mit diesen, z.B. durch Bindung über Wasserstoffbrücken, Bildung von Komplexen oder Einschlüssen.
5) Die Glycerinether wirken durch weitere noch unbekannte Mechanismen, die letztlich zu einer eindeutig überraschenden desodorienden Wirkung beitragen.

Überraschend ist insbesondere, daß beispielsweise 2-Ethylhexylglycerinether trotz der im Vergleich zu bekannten Deo-Wirkstoffen noch guten Wasserlöslichkeit von ca. 0,2 Gew.% einen solch guten desodorierenden Effekt zeigt. Neben der antimikrobiellen Wirksamkeit und den anderen obengenannten möglichen Wirkmechanismen können möglicherweise auch das gute Spreitvermögen, das gute Eindringvermögen in die Haut und/oder die Fähigkeit tiefer in die oberen Schichten der Epidermis einzudringen ausschlaggebend sein.

Die erfindungsgemäßen Glycerinmonoalkylether weisen insbesondere die nachfolgend aufgeführten vorteilhaften Eigenschaften auf:
- gute bis sehr gute Wirkung insbesondere gegen die im Hinblick auf Geruchsverursachung besonders relevanten grampositiven Bakterien
- selektive Wirkung gegen geruchsverursachende Mikroorganismen unter weitgehender Schonung der natürlichen Hautflora
- gutes Haftvermögen auf der Haut
- begrenzt bis wenig wasserlöslich (hinreichend wasserfest)
- ausgezeichnete Stabilität (Hydrolysestabilität, Thermostabilität, pH-Stabilität)
- praktisch nicht flüchtig
- farblos, pH-neutral, inert
- geruchsarm bzw. bei Einsatz einer ausgewählten Qualität spezifisch geruchsneutral
- zum Teil flüssig und daher leicht verarbeitbar, keine Kristallisation in der Zubereitung und auf der Haut
- verträglich mit anderen Wirkstoffen und Hilfsstoffen
- Unterstützung der dispergierenden und coemulgierenden Wirkung in kosmetischen Zubereitungen
- die Glycerinmonoalkyletherstruktur findet sich in zahlreichen natürlich vorkommenden Verbindungen
- aufgrund des breiten Zusatznutzens, den Glycerinmonoalkylether in kosmetischen Präparaten besitzen, kann teilweise auf andere Kosmetikadditive verzichtet werden bzw. deren Menge reduziert werden.

Da die erfindungsgemäßen Glycerinmonoalkylether mild und hautverträglich sind, ein gutes Spreitvermögen besitzen und ein angenehmes Hautgefühl vermitteln, sind sie insbesondere für Deozusammensetzungen geeignet.

Die erfindungsgemäßen Glycerinmonoalkylether können einzeln, in Form eines Gemisches aus zwei oder mehr derselben und darüber hinaus in Kombination mit einem oder mehreren anderen desodorierend wirkenden Stoffen eingesetzt werden. Hierbei handelt es sich vorzugsweise um Antiperspirantien (Adstringentien), antimikrobiell wirkende Stoffe und/oder Duftstoffe. Bevorzugt sind hautverträgliche wasserunlösliche oder begrenzt wasserlösliche (substantive) Wirkstoffe mit spezifischer Wirkung gegen geruchsverursachende Mikroorganismen und/oder natürlich vorkommende desodorierende Wirkstoffe. Als Cowirkstoff speziell geeignet sind natürlich vorkommende, desodorierend wirkende Stoffe wie Farnesol, Phenoxyethanol, Glycerinmonolaurat, Alkalirhodanide, Linalool, Citronellol, Geraniol und Phenethylalkohol, wobei die geruchsarmen Stoffe bevorzugt sind. Beispiele für Wirkstoffe mit geruchsvermindernder Wirkung sind außerdem Aluminiumoxychlorid, Dibromdicyanobutan, 5-Chlor-2-(2,4-dichlorphen-oxy)phenol (Irgasan® DP 300), 2-Brom-2-nitropropandiol-1,3 (Bronopol®), Chlorhexidinsalze, Octenidinsalze, Alexidinsalze sowie Salze anderer kationenaktiver Verbindungen mit desodorierender Wirkung, wobei die Rhodanidsalze bevorzugt sind. Insbesondere bevorzugt sind Farnesol und Phenoxyethanol.

Bei Verwendung einer Kombination der Glycerinmonoalkylether mit solchen anderen Deowirkstoffen/Antiperspirantien werden zum Teil auch synergistische Wirkungssteigerungen beobachtet.

Die erfindungsgemäßen Glycerinmonoalkylether werden bezogen auf die gebrauchsfertige Deozusammensetzung in einer Konzentration im Bereich von 0,01 bis 20 Gew.% eingesetzt. Vorzugsweise liegt die Konzentration im Bereich von 0,1 bis 15 Gew.% und insbesondere im Bereich von 0,5 bis 10 Gew.%.

Das Verhältnis von Glycerinmonoalkylether zu dem/den anderen desodorierend wirkenden Zusammensetzungsbestandteilen liegt im Bereich von 50:1 bis 1:50, vorzugsweise 10:1 bis 1:10 und insbesondere bevorzugt im Bereich von 5:1 bis 1:1.

In der fertigen Gebrauchslösung liegen die erfindungsgemäßen Deowirkstoffe in einem wäßrigen und/oder alkoholischen Medium vor. Die zur Bildung des alkoholischen Mediums bzw. der alkoholischen Komponente des Mediums geeigneten Alkohole schließen C₂-C₃-Alkanole, Glykole und Polyglykole ein. Insbesondere umfassen die C₂-C₃-Alkanole Ethanol, Propanol, Isopropanol und/oder Mischungen derselben und die Polyglykole umfassen Polyethylenglykol, Polypropylenglykol und/oder Mischungen derselben. Gemische aus Glycerinmonoalkylethern und niederen Alkoholen wie beispielsweise Ethanol besitzen eine ausgeprägte synergistische antimikrobielle Wirksamkeit, die, da Ethanol häufig als Bestandteil von Deoformulierungen verwendet werden, zusätzlich ausgenutzt werden könne. Im übrigen können die Alkohole als Lösungsvermittler dienen. Glykole und Polyglykole weisen demgegenüber keine erfindungsgemäße ausnutzbaren antimikrobiellen Eigenschaften auf.

Die Erfindung betrifft neben der Verwendung der erfindungsgemäßen Glycerinmonoalkylether (a) allein oder in Kombination mit anderen desodorierend wirkenden Stoffen ferner die entsprechenden Zusammensetzungen aus Glycerinmonoalkylether und einem oder mehreren anderen desodorierenden wirkenden Stoffen ausgewählt aus Adstringentien, hautverträglichen wasserunlöslichen oder begrenzt wasserlöslichen (substantiven) Wirkstoffen mit spezifischer Wirkung gegen geruchsverursachende Mikroorganismen und/oder natürlich vorkommenden desodorierenden Wirkstoffen, ausgenommen Kombinationen von
(i) 1-Monoisostearylglycerylether mit Glycerylmonooleat oder Glycerylmonolaurat,
(ii) Glycerylmonoalkylethern gemäß a) mit aromatischen Alkoholen der Formel 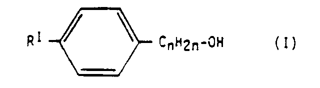 in der R¹ Wasserstoff oder eine Alkylgruppe mit 1 bis 4 Kohlenstoffatomen ist und n eine ganze Zahl von 1 bis 6 ist, und
(iii) Glycerylmonoalkylethern gemäß a) mit C₁-C₆-Alkylalkoholen, wenn keine anderen desodorierend wirkenden Stoffe vorhanden sind.
Hierbei sind insbesondere Zusammensetzungen bevorzugt, die neben Glycerinmonoalkylether Farnesol, Phenoxyethanol oder Glycerinmonolaurat enthalten.

Diese Zusammensetzungen werden hergestellt, indem die Einzelkomponenten gegebenenfalls unter Erwärmen miteinander vermischt werden.

Die Erfindung ist im folgenden anhand von Ausführungsbeispielen näher erläutert.

Die in der folgenden Tabelle angegebenen Formulierungen wurden im Agarlochtest auf Wirksamkeit gegenüber Deokeimen geprüft, wobei die mikrobiologische Wirksamkeit als Durchmesser des jeweiligen Hemmhofs in mm angegeben ist.

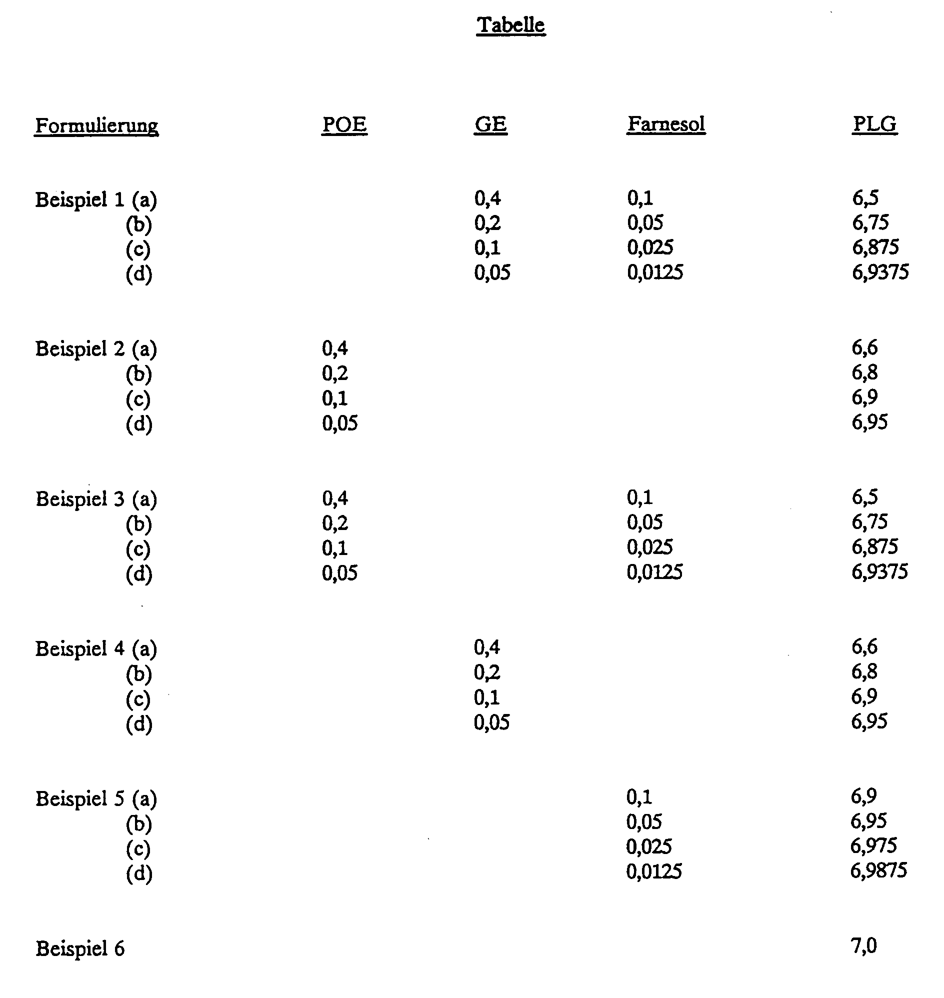

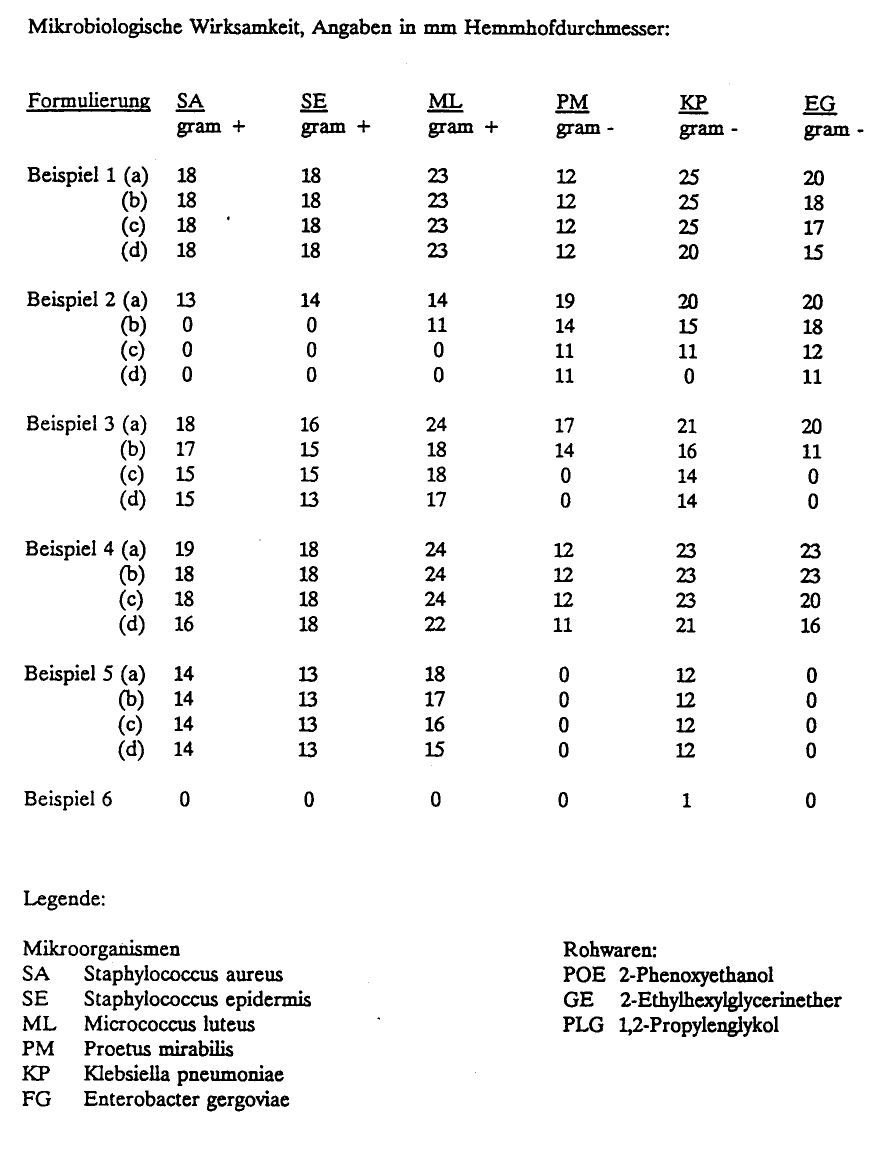

### Platten-Diffusionstest

### 1. Agarlochtest

- Bakterien: 18 - 24 Stunden Bouillon-Kulturen werden 1:4 verdünnt (1 ml Bakteriensuspension + 3 ml CSL).
   Hefen: eine 4 Tage alte Candida albicans-Kultur (CSA + Traubenzucker) wird mit 5 ml physiologischer Kochsalzlösung abgeschwemmt und nach einem Bariumsulfat-Standard eingestellt.
- Von der Bakteriensuspension werden 0,2 ml auf CSA ausgespatelt.
   Von der Candida albicans-Suspension werden 0,1 ml auf CSA ausgespatelt.
- In die Mitte der Agarplatten wird mit einem abgeflammten Korkbohrer ein Loch von 8 mm Durchmesser gestanzt.
- Dieses Loch wird bis zum Rand mit dem zu prüfenden Präparat gefüllt.
- Die Platten werden zwei Stunden bei Raumtemperatur zum Vordiffundieren stehengelassen.
- Bakterien und Hefe werden 24 - 48 Stunden bei 37°C bebrütet.
- Auswertung: Hemmhof in mm vom Rand des Loches ausgehend

- CSA =: Caseinpepton-Sojamehlpepton-Agar
- CSL =: Caseinpepton-Sojamehlpepton-Lösung

Aus den Ergebnissen des Agarloch-Hemmhoftestes lassen sich folgende Aussagen bezüglich der Deowirksamkeit der Zubereitungen ableiten:
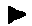 2-Ethylhexylglycerinether besitzen eine gute Wirksamkeit als Deowirkstoff, wobei die Wirksamkeit gegenüber den für die Geruchsverursachung besonders relevanten gram-positiven Bakterien gegeben ist.
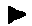 2-Ethylhexylglycerinether ist deutlich wirksamer als Farnesol.
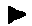 2-Ethylhexylglycerinether in Kombination mit Farnesol ist wirksamer als Farnesol allein und ist wirksamer als eine Kombination aus Phenoxyethanol und Farnesol.
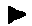 Phenoxyethanol allein ist wegen mangelnder Wirksamkeit praktisch nicht als Deowirkstoff geeignet.
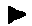 Während das Wirkungsspektrum von Phenoxyethanol gegenüber gram-positiven und gram-negativen Keimen eher ausgeglichen ist, wirkt 2-Ethylhexylglycerinether eher spezifisch gegen gram-positive Keime.

Es wurde eine Formulierung bestehend aus
- 1,0 Teile: 2-Ethylhexylglycerinether
- 99,0 Teile: Ethanol, vergällt
verwendet, um die Eignung der erfindungsgemäßen Glycerinether als Deo-Wirkstoffe im praxisgerechten Versuch zu testen. Es wurde gefunden, daß diese Formulierung bei praxisüblicher Verwendung als Deodorant über einen Zeitraum von bis zu 2 Monaten von mehreren Personen als wirksam, angenehm in der Anwendung und z.T. durchaus besser wirkt als handelsübliche Deodorantien. Bei einmaliger morgendlicher Anwendung wurde ein Schutz vor Geruchsentwicklung über den ganzen Tag und noch darüber hinaus beobachtet.

Die Eignung der erfindungsgemäßen Glycerinether als Deo-Wirkstoff in Kombination mit anderen Deowirkstoffen ist auch anhand des sogenannten Sniffing-Tests untersucht worden. Hierbei wurden Wirkstoffformulierungen untersucht, die 2-Ethylglycerinether in Kombination mit anderen Deo-Wirkstoffen oder nur andere Deo-Wirkstoffe enthalten. Es wurde im Paarvergleich getestet. Die Testmethode ist in "dragoco report" 6/76 beschrieben.
1. In den Test werden 20 gesunde männliche und weibliche Erwachsene einbezogen, von denen bekannt ist, daß ein Problem mit Schweißgeruch vorliegt. Die Testpersonen werden angewiesen, vor Beginn der Prüfung über einen Zeitraum von 10 Tagen eine unparfümierte Seife ohne antibakterizide Wirkstoffe unter den Achselhöhlen anzuwenden. Während des gesamten Testzeitraumes wird in dieser Körperregion keine Fremdkosmetik verwendet. Lediglich die unparfümierte Seife wird einmal täglich angewendet.
2. Am 11. Tag, 5 Stunden nach dem letzten Waschvorgang, werden die Achselhöhlen von 3 Testern auf Geruch berochen (besnifft) und nach Noten von 0 bis 5 beurteilt:
   - 0 =: kein Schweißgeruch
   - 1 =: sehr geringer Schweißgeruch
   - 2 =: geringer Schweißgeruch
   - 3 =: mäßiger Schweißgeruch
   - 4 =: starker Schweißgeruch
   - 5 =: sehr starker Schweißgeruch.
   Entscheidend für das Urteil ist der Paarvergleich (linke gegen rechte Achsel) beim jeweiligen Probanden. Wahrnehmbare paarweise Unterschiede werden mit mindestens einer Note Differenz beurteilt. Die Ausgangswerte dürfen nicht geringer als Note 3 ausfallen.
3. Danach werden die Deoformulierungen an die Probanden mit der Auflage ausgegeben, die Produkte über einen Zeitraum von 5 Tagen standardisiert anzuwenden. 10 Probanden applizieren DEO-A unter der rechten und Deo-B, C oder D unter der linken Achselhöhle, 10 Probanden applizieren genau entgegengesetzt.
4. Am 5. Produktanwendungstag, 5 Stunden nach der letzten Anwendung, wird erneut besnifft und beurteilt.
5. Um praxisgerechte Ergebnisse zu erzielen, wird keine Standardisierung der Kleidung vorgenommen.

### Auswertung

Die Einzelnoten wurden gemittelt und die Standardabweichung berechnet. Um zu prüfen, ob zwischen den paarig geprüften Deoformulierungen ein Unterschied besteht bzw. ob zwischen Ausgangs- und Endwert jeder Formulierung eine Differenz nachweisbar ist, wurde der paarweise t-Test (Lothar Sachs, Angewandte Statistik, Springer Verlag, 6. Auflage, 1984, Seite 242 - 244) durchgeführt und mit dem Vorzeichentest von Dixon und Mood (Lothar Sachs, Angewandte Statistik, Springer Verlag, 6. Auflage, 1984, Seite 247 - 250) überprüft. Zum Vergleich der paarweise geprüften Produkte wurden die paarigen Einzelwertdifferenzen verwendet.

### Deo-Formulierungen für den Sniff-Test

| | A | B | C | D |
|---|---|---|---|---|
| Irgasan® DP 300 | 0,1 | | | |
| Dibromdicyanobutan | | 0,015 | | |
| Quartamin-Wikstoff* | | | 0,2 | |
| Natriumthiocyanat | | | 0,038 | |
| Octenidindihydrochlorid | | | | 0,1 |
| Phenoxyethanol | | 0,15 | 1,762 | |
| 2-Ethyhexylglycerinether | | 0,135 | | 0,9 |
| 1,2-Propylenglykol | 1,0 | 1,0 | 1,0 | 1,0 |
| Ethanol, DEP-verg. | 40,0 | 40,0 | 40,0 | 40,0 |
| VE-Wasser | 58,9 | 58,7 | 57,0 | 58,0 |

| | | | | |
|---|---|---|---|---|
| * N-Benzyl-N-(2-hydroxyethyl)-N-(lauroyloxyethyl)-N-methylammoniumchlorid | | | | |

### Ergebnisse:

| Produktvergleich | mittlere Ausgangswerte | | mittlere Endwerte | | bemerkbarer Unterschied |
|---|---|---|---|---|---|
| | A | B, C u. D | A | B, C u. D | |
| Deo-A/Deo-B | 4,03 | 4,08 | 3,28 | 3,00 | ja |
| Deo-A/Deo-C | 3,98 | 3,90 | 2,90 | 2,93 | nein |
| Deo-A/Deo-D | 3,76 | 3,92 | 2,89 | 2,63 | ja |

Alle Produkte im Test auch Deo-A bewirken eine Reduzierung des Schweißgeruchs im Axillenbereich nach 5 Tagen Anwendung und 5 Stunden Anwendungspause. Es gibt deutliche Anzeichen dafür, daß Deo-B und Deo-D eine bessere Deowirkung aufweisen als DEo-A. Zwischen Deo-A und Deo-C bestehen hinsichtlich der Deowirkung keine wesentlichen Unterschiede.

## Patentansprüche

1. Deo-Zusammensetzung, dadurch gekennzeichnet, daß sie eine Kombination
a) eines oder mehrerer Glycerinmonoalkylether der allgemeinen Formel
R - O - CH₂ - CHOH - CH₂OH ,
in der R eine verzweigte oder unverzweigte C₆-C₁₈-Alkylgruppe ist, wobei die Alkylgruppe mit einer oder mehreren Hydroxy- und/oder C₁-C₄-Alkoxygruppe(n) substituiert und/oder die Alkylkette durch bis zu vier Sauerstoffatome unterbrochen sein kann, d.h. Alkylenoxygruppen wie Ethylenoxy- und Propylenoxygruppen enthalten kann, mit
b) einem oder mehreren anderen desodorierenden wirkenden Stoffen ausgewählt aus Adstringentien, hautverträglichen wasserunlöslichen oder begrenzt wasserlöslichen (substantiven) Wirkstoffen mit spezifischer Wirkung gegen geruchsverursachende Mikroorganismen und/oder natürlich vorkommenden desodorierenden Wirkstoffen umfaßt,
ausgenommen Kombinationen von
(i) 1-Monoisostearylglycerylether mit Glycerylmonooleat oder Glycerylmonolaurat,
(ii) Glycerylmonoalkylethern gemäß a) mit aromatischen Alkoholen der Formel 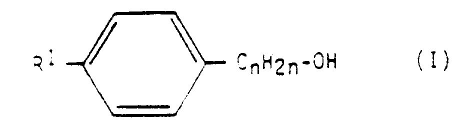 in der R¹ Wasserstoff oder eine Alkylgruppe mit 1 bis 4 Kohlenstoffatomen ist und n eine ganze Zahl von 1 bis 6 ist, und
(iii) Glycerylmonoalkylethern gemäß a) mit C₁-C₆-Alkylalkoholen, wenn keine anderen desodorierend wirkenden Stoffe vorhanden sind.

2. Zusammensetzung nach Anspruch 1, dadurch gekennzeichnet, daß es sich bei den natürlich vorkommenden desodorierenden Wirkstoffen um Farnesol, Phenoxyethanol, Glycerinmonolaurat, Alkalirhodanide, Linalool, Citronellol oder Geraniol handelt.

3. Zusammensetzung nach Anspruch 1 oder 2, dadurch gekennzeichnet, daß es sich bei den desodorierenden Wirkstoffen um Aluminiumoxychlorid, Dibromdicyanbutan, 5-Chlor-2-(2,4-dichlorphenoxy)phenol, 2-Brom-2-nitropropandiol-1,3, Chlorhexidinsalze, Octenidinsalze, Alexidinsalze sowie Salze anderer kationenaktiver Verbindungen mit desodorierender Wirkung handelt, insbesondere Rhodanidsalze.

4. Zusammensetzung nach einem der Ansprüche 1 bis 3, dadurch gekennzeichnet, daß sie die Wirkstoffkombination in einer Konzentration im Bereich von 0,01 bis 20 Gew.%, insbesondere 0,1 bis 15 Gew.% und bevorzugt 0,5 bis 10 Gew.% enthält.

5. Zusammensetzung nach einem der Ansprüche 1 bis 4, dadurch gekennzeichnet, daß das Verhältnis von Glycerinmonoalkylether zu dem/den anderen desodorierenden Stoffen im Bereich von 50:1 bis 1:50, insbesondere 10:1 bis 1:10 liegt.

6. Zusammensetzung nach einem der Ansprüche 1 bis 5, dadurch gekennzeichnet, daß die Deo-Wirkstoffe in wäßrigem und/oder alkolischem Medium vorliegen, wobei das alkoholische Medium insbesondere C₂-C₃-Alkanole, bevorzugt Ethanol, Propanol, Isopropanol und/oder Mischungen derselben, Glykole und Polyglykole, bevorzugt Polyethylenglykol, Polypropylenglykol und/oder Mischungen derselben umfaßt.

7. Verwendung von Glycerinmonoalkylethern der allgemeinen Formel
R - O - CH₂ - CHOH - CH₂OH ,
in der R eine verzweigte oder unverzweigte C₆-C₁₈-Alkylgruppe, insbesondere eine C₈-C₁₂-Alkylgruppe ist, wobei die Alkylgruppe mit einer oder mehreren Hydroxy- und/oder C₁-C₄-Alkoxygruppe(n) substituiert und/oder die Alkylkette durch bis zu vier Sauerstoffatome unterbrochen sein kann, d.h. Alkylenoxygruppen wie Ethylenoxy- und Propylenoxygruppen enthalten kann, einzeln oder in Form eines Gemisches aus zwei oder mehr derselben als Deo-Wirkstoffe zur Unterbindung von unangenehmen Gerüchen,
ausgenommen Kombinationen von 1-Monoisostearylglycerylether mit Glycerylmonooleat oder Glycerylmonolaurat.

8. Verwendung nach Anspruch 7, dadurch gekennzeichnet, daß R eine 2-Ethylhexylgruppe oder Dodecylgruppe und insbesondere eine 2-Ethylhexylgruppe ist.

9. Verwendung nach Anspruch 7 oder 8, dadurch gekennzeichnet, daß eine Kombination eines oder mehrerer der Glycerinmonoalkylether mit einem oder mehreren anderen desodorierenden wirkenden Stoffen eingesetzt wird, wobei es sich bei den anderen desodorierend wirkenden Stoffen insbesondere um Antiperspirantien (Adstringentien), antimikrobiell wirkende Stoffe und/oder Duftstoffe handelt und bevorzugt hautverträgliche wasserunlösliche oder begrenzt wasserlösliche (substantive) Wirkstoffe mit spezifischer Wirkung gegen geruchsverursachende Mikroorganismen und/oder natürlich vorkommende desodorierende Wirkstoffe handelt.

10. Verwendung nach Anspruch 9, dadurch gekennzeichnet, daß es sich bei den natürlich vorkommenden desodorierenden Wirkstoffen um Farnesol, Phenoxyethanol, Glycerinmonolaurat, Alkalirhodanide, Linalool, Citronellol, Geraniol, Phenethylalkohol handelt.

11. Verwendung nach Anspruch 9 oder 10, dadurch gekennzeichnet, daß es sich bei den desodorierenden Wirkstoffen um Aluminiumoxychlorid, Dibromdicyanbutan, 5-Chlor-2-(2,4-dichlorphenoxy)phenol, 2-Brom-2-nitropropandiol-1,3, Chlorhexidinsalze, Octenidinsalze, Alexidinsalze sowie Salze anderer kationenaktiver Verbindungen mit desodorierender Wirkung handelt, insbesondere Rhodanidsalze.

12. Verwendung nach einem der Ansprüche 7 bis 11, dadurch gekennzeichnet, daß der/die Glycerinmonoalkylether in einer Konzentration im Bereich von 0,01 bis 20 Gew.%, insbesondere 0,1 bis 15 Gew.% und bevorzugt 0,5 bis 10 Gew.% eingesetzt werden.

13. Verwendung nach einem der Ansprüche 9 bis 12, dadurch gekennzeichnet, daß das Verhältnis von Glycerinmonoalkylether zu dem/den anderen desodorierenden Stoffen im Bereich von 50:1 bis 1:50, insbesondere 10:1 bis 1:10 liegt.

14. Verwendung nach einem der Ansprüche 7 bis 13, dadurch gekennzeichnet, daß die Deo-Wirkstoffe in wäßrigem und/oder alkolischem Medium eingesetzt werden, wobei das alkoholische Medium insbesondere C₂-C₃-Alkanole, bevorzugt Ethanol, Propanol, Isopropanol und/oder Mischungen derselben, Glykole und Polyglykole, bevorzugt Polyethylenglykol, Polypropylenglykol und/oder Mischungen derselben umfaßt.

## Claims

1. Deo-composition characterized in that it comprises a combination
a) of one or more glycerin monoalkyl ethers of the general formula
R - O - CH₂ - CHOH - CH₂OH,
in which R is a branched or unbranched C₆-C₁₈ alkyl group, whereby the alkyl group can be substituted by one or more hydroxy and/or C₁-C₄ alkoxy group(s) and/or the alkyl chain can be interrupted by up to four oxygen atoms, i.e. can contain alkylenoxy groups such as ethylenoxy and propylenoxy groups, with
b) one or more other deodorizing active substances selected from astringents, skin-compatible water-insoluble or limitedly water-soluble (substantive) active ingredients with specific action against odour-causing microorganisms and/or naturally occurring deodorizing active ingredients,
with the exception of combinations of
(i) 1-monoisostearylglyceryl ether with glyceryl monooleate or glyceryl monolaurate,
(ii) glyceryl monoalkyl ethers in accordance with a) with aromatic alcohols of the formula 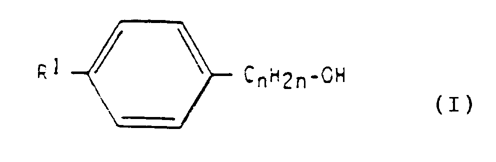 in which R¹ is hydrogen or an alkyl group with 1 to 4 carbon atoms and n is a whole number from 1 to 6, and
(iii) glyceryl monoalkyl ethers in accordance with a) with C₁-C₆ alkyl alcohols, if no other substances having a deodorizing action are present.

2. Composition according to Claim 1, characterized in that the naturally occurring deodorizing active ingredients are farnesol, phenoxyethanol, glycerin monolaurate, alkali rhodanides, linalool, citronellol or geraniol.

3. Composition according to Claim 1 or 2, characterized in that the deodorizing active ingredients are aluminium oxychloride, dibromodicyanobutane, 5-chloro-2-(2,4-dichlorophenoxy)phenol, 2-bromo-2-nitropropanediol-1,3, chlorohexidine salts, octenidine salts, alexidine salts and salts of other cationic compounds having a deodorizing action, in particular rhodanide salts.

4. Composition according to one of Claims 1 to 3, characterized in that it contains the active ingredient combination in a concentration in the range from 0.01 to 20 wt.%, in particular 0.1 to 15 wt.% and preferably 0.5 to 10 wt.%.

5. Composition according to one of Claims 1 to 4, characterized in that the ratio of glycerin monoalkyl ether to the other deodorizing substance(s) lies in the range from 50:1 to 1:50, in particular 10:1 to 1:10.

6. Composition according to one of Claims 1 to 5, characterized in that the deo-active ingredients are present in aqueous and/or alcoholic medium, whereby the alcoholic medium comprises in particular C₂-C₃ alkanols, preferably ethanol, propanol, isopropanol and/or mixtures thereof, glycols and polyglycols, preferably polyethylene glycol, polypropylene glycol and/or mixtures thereof.

7. Use of glycerin monoalkyl ethers of the general formula
R - O - CH₂ - CHOH - CH₂OH,
in which R is a branched or unbranched C₆-C₁₈ alkyl group, in particular a C₈-C₁₂ alkyl group, whereby the alkyl group can be substituted by one or more hydroxy and/or C₁-C₄ alkoxy group(s) and/or the alkyl chain can be interrupted by up to four oxygen atoms, i.e. can contain alkylenoxy groups such as ethylenoxy and propylenoxy groups, individually or in the form of a mixture of two or more of the same, as deo-active ingredients for preventing unpleasant odours,
with the exception of combinations of 1-monoisostearylglyceryl ether with glyceryl monooleate or glyceryl monolaurate.

8. Use according to Claim 7, characterized in that R is a 2-ethylhexyl group or dodecyl group and in particular a 2-ethylhexyl group.

9. Use according to Claim 7 or 8, characterized in that a combination of one or more of the glycerin monoalkyl ethers with one or more other deodorizing active substances is used, the other substances having a deodorizing action being in particular antiperspirants (astringents), substances with an anti-microbial action and/or fragrances and being preferably skin-compatible water-insoluble or limitedly water-soluble (substantive) active ingredients with a specific action against odour-causing microorganisms and/or naturally occurring deodorizing active ingredients.

10. Use according to Claim 9, characterized in that the naturally occurring deodorizing active ingredients are farnesol, phenoxyethanol, glycerin monolaurate, alkali rhodanides, linalool, citronellol, geraniol, phenethyl alcohol.

11. Use according to Claim 9 or 10, characterized in that the deodorizing active ingredients are aluminium oxychloride, dibromodicyanobutane, 5-chloro-2-(2,4-dichlorophenoxy)phenol, 2-bromo-2-nitropropanediol-1,3, chlorohexidine salts, octenidine salts, alexidine salts and salts of other cationic compounds having a deodorizing action, in particular rhodanide salts.

12. Use according to one of Claims 7 to 11, characterized in that the glycerin monoalkyl ether(s) is (are) used in a concentration in the range from 0.01 to 20 wt.%, in particular 0.1 to 15 wt.% and preferably 0.5 to 10 wt.%.

13. Use according to one of Claims 9 to 12, characterized in that the ratio of glycerin monoalkyl ether to the other deodorizing substance(s) lies in the range from 50:1 to 1:50, in particular 10:1 to 1:10.

14. Use according to one of Claims 7 to 13, characterized in that the deo-active ingredients are used in aqueous and/or alcoholic medium, whereby the alcoholic medium comprises in particular C₂-C₃ alkanols, preferably ethanol, propanol, isopropanol and/or mixtures thereof, glycols and polyglycols, preferably polyethylene glycol, polypropylene glycol and/or mixtures thereof.

## Revendications

1. Composition déodorante, caractérisée en ce qu'elle comprend une combinaison
a) d'un ou de plusieurs monoalkyléthers de glycérine de formule générale
R - O - CH₂ - CHOH - CH₂OH,
dans laquelle R est un groupe alkyle en C₆ à C₁₈ linéaire ou ramifié, le groupe alkyle pouvant être substitué par un ou plusieurs groupes hydroxy et/ou alcoxy en C₁ à C₄ et/ou la chaîne alkyle pouvant être interrompue par jusqu'à 4 atomes d'oxygène, c'est-à-dire qu'elle peut contenir des groupes alkylènoxy, tels qu'éthylènoxy et propylènoxy, avec
b) une ou plusieurs autres substances désodorisantes choisies parmi des substances astringentes, des principes actifs (substantifs) tolérés par la peau, insolubles dans l'eau ou dont la solubilité dans l'eau est limitée, à activité spécifique contre les micro-organismes causant les odeurs, et/ou des principes actifs désodorisants naturels,
à l'exception des combinaisons de
(i) éther de 1-monoisostéaryle et de glycéryle avec du monooléate de glycéryle ou du monolaurate de glycéryle,
(ii) monoalkyléthers de glycéryle selon a) avec des alcools aromatiques de formule 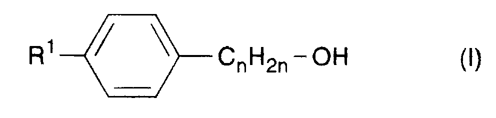 dans laquelle R¹ est un atome d'hydrogène ou un groupe alkyle à 1 à 4 atomes de carbone et n un nombre entier de 1 à 6, et
(iii) monoalkyléthers de glycéryle selon a) avec des alkylalcools en C₁ à C₆, lorsqu'aucune autre substance désodorisante n'est présente.

2. Composition selon la revendication 1, caractérisée en ce que, pour les principes actifs désodorisants naturels, il s'agit de farnésol, de phénoxyéthanol, de monolaurate de glycérine, de thiocyanates alcalins, de linalol, de citronellol ou de géraniol.

3. Composition selon la revendication 1 ou 2, caractérisée en ce qu'il s'agit, pour les principes actifs désodorisants, d'oxychlorure d'aluminium, de dibromodicyanobutane, de 5-chloro-2-(2,4-dichlorophénoxy)phénol, de 2-bromo-2-nitropropane-1,3-diol, de sels de la chlorhéxidine, de sels d'octénidine, de sels d'alexidine, ainsi que de sels d'autres composés à activité de cations à action désodorisante, en particulier des sels thiocyanates.

4. Composition selon une des revendications 1 à 3, caractérisée en ce que la combinaison de principes actifs est contenue dans une concentration comprise dans un domaine de 0,01 à 20 % en poids, en particulier de 0,1 à 15 % en poids et de préférence de 0,5 à 10% en poids.

5. Composition selon une des revendications 1 à 4, caractérisée en ce que la proportion de monoalkyléther de glycérine et de l'autre (des autres) substance(s) désodorisante(s) est comprise dans un domaine de 50/1 à 1/50, en particulier de 10/1 à 1/10.

6. Composition selon une des revendications 1 à 5, caractérisée en ce que les principes actifs désodorisants sont dans un milieu aqueux et/ou alcoolique, le milieu alcoolique comprenant en particulier des alcanols en C₂ à C₃, de préférence de l'éthanol, du propanol, de l'isopropanol et/ou des mélanges des alcools cités, des glycols et des polyglycols, de préférence du polyéthylène glycol, du polypropylène glycol et/ou des mélanges de ceux-ci.

7. Utilisation de monoalkyléthers de glycérine de formule générale
R - O - CH₂ - CHOH - CH₂OH,
dans laquelle R est un groupe alkyle en C₆ à C₁₈ linéaire ou ramifié, en particulier un groupe alkyle en C₈ à C₁₂, le groupe alkyle pouvant être substitué par un ou plusieurs groupes hydroxy et/ou alcoxy en C₁ à C₄ et/ou la chaîne alkyle pouvant être interrompue par jusqu'à 4 atomes d'oxygène, c'est-à-dire qu'elle peut contenir des groupes alkylènoxy, tels qu'éthylènoxy et propylènoxy, seuls ou sous forme d'un mélange de deux composés ou plus, comme principe actif désodorisant pour arrêter les mauvaises odeurs,
à l'exception des combinaisons d'éther de 1-monoisostéaryle et de glycéryle avec du monooléate de glycéryle ou du monolaurate de glycéryle.

8. Utilisation selon la revendication 7, caractérisée en ce que R est un groupe 2-éthylhexyle ou un groupe dodécyle et en particulier un groupe 2-éthylhexyle.

9. Utilisation selon la revendication 7 ou 8, caractérisée en ce que l'on utilise une combinaison d'un ou de plusieurs des monoalkyléthers de glycérine avec une ou plusieurs autres substances désodorisantes, s'agissant pour ces autres principes actifs désodorisants en particulier d'antitranspirants (astringents), de principes actifs à action antimicrobienne et/ou d'agents odorants et s'agissant de préférence de principes actifs (substantifs) insolubles dans l'eau ou dont la solubilité est limitée, tolérés par la peau, à action spécifique contre les micro-organismes causant les odeurs et/ou de principes actifs désodorisants naturels.

10. Utilisation selon la revendication 9, caractérisée en ce qu'il s'agit, pour les principes actifs désodorisants naturels, de farnésol, de phénoxyéthanol, de monolauratede glycérine, de thiocyanates alcalins, de linalol, de citronellol, de géraniol, de phénéthylalcool.

11. Utilisation selon la revendication 9 ou 10, caractérisée en ce qu'il s'agit, pour les principes actifs désodorisants, d'oxychlorure d'aluminium, de dibromodicyanobutane, de 5-chloro-2-(2,4-dichlorophénoxy)phénol, de 2-bromo-2-nitropropane-1,3-diol, de sels de la chlorhéxidine, de sels d'octénidine, de sels d'alexidine, ainsi que de sels d'autres composés à activité de cations à action désodorisante, en particulier des sels thiocyanates.

12. Utilisation selon une des revendications 7 à 11, caractérisée en ce que le (les) monoalkyléther(s) de glycérine est (sont) utilisé(s) en une concentration comprise dans un domaine de 0,01 à 20 % en poids, en particulier de 0,1 à 15% en poids et de préférence de 0,5 à 10 % en poids.

13. Utilisation selon une des revendications 9 à 12, caractérisée en ce que la proportion de monoalkyléther de glycérine et de l'autre (des autres) substance(s) désodorisante(s) est comprise dans un domaine de 50/1 à 1/50, en particulier de 10/1 à 1/10.

14. Utilisation selon une des revendications 7 à 13, caractérisée en ce que les principes actifs désodorisants sont utilisés dans un milieu aqueux et/ou alcoolique, le milieu alcoolique comprenant en particulier des alcanols en C₂ à C₃, de préférence l'éthanol, le propanol l'isopropanol et/ou des mélanges des alcools cités, des glycols et des polyglycols, de préférence du polyéthylène glycol, du polypropylène glycol et/ou des mélanges de ceux-ci.
